Europäisches Patentamt

European Patent Office

Office européen des brevets

(19)

(11) Veröffentlichungsnummer: **0 030 583**
**B1**

(12) **EUROPÄISCHE PATENTSCHRIFT**

(45) Veröffentlichungstag der Patentschrift: **13.06.84**

(21) Anmeldenummer: **79810183.8**

(22) Anmeldetag: **18.12.79**

(51) Int. Cl.³: **A 61 F 1/00**, A 61 L 17/00,
A 61 K 37/12

(54) Knochenersatzmaterial und Verfahren zur Herstellung eines Knochenersatzmaterials.

(43) Veröffentlichungstag der Anmeldung:
**24.06.81 Patentblatt 81/25**

(45) Bekanntmachung des Hinweises auf die Patenterteilung:
**13.06.84 Patentblatt 84/24**

(84) Benannte Vertragsstaaten:
**CH DE FR IT**

(56) Entgegenhaltungen:
EP - A - 0 018 496
DE - A - 2 022 498
FR - A - 2 350 826
FR - A - 2 374 040
FR - A - 2 410 477
US - A - 3 523 807
US - A - 4 064 567

(73) Patentinhaber: **OSTEO AG**
**Bielstrasse 620**
**CH-2545 Selzach (CH)**

(72) Erfinder: **Mittelmeier, Heinz, Prof.**
**An Der Schutzhütte**
**D-Blieskastel (DE)**
Erfinder: **Moser, Heinz**
**Spåretweg 440**
**CH-2545 Selzach (CH)**
Erfinder: **Leu, Beat**
**Kirschbaumallee 14**
**CH-2563 Ipsach (CH)**

(74) Vertreter: **Seehof, Michel**
**c/o AMMANN PATENTANWAELTE AG BERN**
**Schwarztorstrasse 31**
**CH-3001 Bern (CH)**

Courier Press, Leamington Spa, England.

## Beschreibung

Die vorliegende Erfindung bezieht sich auf ein Knochenersatzmaterial und auf ein Verfahren zur Herstellung desselben.

In der orthopädischen Chirurgie gibt es zahlreiche Erkrankungen oder Verletzungsfolgen mit Skelettdefekten, zu deren Wiederherstellung Knochenersatzmaterial benötigt wird. Dies beispielsweise infolge von gutartigen zystischen Tumoren, die zu grossen Knochenhöhlen mit der Gefahr von Spontanfrakturen führen, Knochendefekte nach Resektion bösartiger Tumoren, Knochenlücken nach Verlängerungsosteotomie und Defektpseudarthrosen oder zur Erzielung von ausgedehnten Versteifungen der Wirbelsäule.

Als Knochenersatzmaterial hat sich am besten autologes Knochenmaterial bewährt, das an einer anderen Stelle, beispielsweise vom Becken oder Schienbein aus dem Körper entfernt und im Bereich des Defektes eingepflanzt wird. Das autologe Knochenmaterial hat vor allem den Vorteil, dass es als individualgleiches Eiweissmaterial ohne immunologische Abwehrreaktion rasch eingebaut wird. Andererseits hat die autologe Knochenplastik den Nachteil, dass eine weitere Wunde gesetzt wird, welche den Patienten biologisch zusätzlich belastet (Blutverlust) und selbst einen Defekt hinterlässt, der sich nut langsam schliesst und manchmal auch eine Strukturschwäche des betreffenden Skelettabschnittes hinterlässt. Aus diesem Grunde hat man versucht, diesen Nachteil zu umgehen und sich der Homoioplastik bedient, wobei Knochenmaterial von anderen Menschen verwendet wird, das von Leichen gewonnen wird. Dieses Material wird bis zur Verwendung entweder in sterilhaltenden Salzlösungen, zum Beispiel Zialit oder im Tiefkühlverfahren konserviert. Bei der Homoioplastik handelt es sich um ein artgleiches, jedoch individualverschiedenes Eiweiss, das zu gewissen immunologischen Abwehrreaktionen führt und infolgedessen keinen so guten osteoplastischen Effekt wie autologes Material aufweist. Es regt zwar zu Knochenneubildung an; dies ist jedoch durch die Notwendigkeit biologischer Aufbauprozesse des Transplantates behindert. Ausserdem stellt die sterile Gewinnung und Lagerhaltung des Materials eine gewisses Problem und eine Belastung für die betreffenden Kliniken dar. Ferner entstehen bei einer solchen Organentnahme bestimmte gesetzliche Probleme.

Seit langem hat man sich auch bemüht, artfremdes Knochenmaterial von Tieren zu verwenden, an das man relativ leicht herankommt. Jedoch führt die Artverschiedenheit des Eiweisses bei dieser Heteroplastik zu stärkeren immunologischen Abwehrreaktionen mit Abstossung oder Abkapselung des Transplantates und nur spärlicher Anregung zur Knochenneubildung.

Es ist auch schon bekannt, heteroplastisches, d.h. tierisches Knochenmaterial in Form von Knochenspänen oder Knochenmehl (Corticalis und Spongiosa) zu verwenden, wobei vor allem Material vom Kalb zur Anwendung gelangt. Bei diesem Material sind die Eiweisssubstanzen des Knochenmarks ausgewaschen und der Knochen ist entfettet und enteiweisst. Allerdings enthält dieses Material noch einen gewissen Prozentsatz Kollagen und Apatit. Dieses unter dem Namen "Kieler Knochenspan" bekannte Material konnte sich jedoch nicht durchsetzen, so dass heute grösstenteils auf den autologen oder homologen Knochenersatz zurückgegriffen wird.

Aus dem älteren Recht, der EP—A—0018496, ist ein chirurgisches Material auf Basis von chirurgischem Fadenmaterial bekannt, das aus resorbierbaren oder nichtresorbierbaren Fäden besteht, die ein Netzwerk bilden. Ausserdem können die resorbierbaren Polymerfäden mit Tricalciumphosphat bestückt sein, wobei die Fäden auch aus natürlichem Kollagen bestehen können. Dieses natürliche Kollagen kann jedoch immunologische Abwehrreaktionen hervorrufen, während das resorbierbare Tricalciumphosphat beim Abbau desselben eine Schwächung verursacht, bis der an- und einwachsende Knochen eine genügende Festigkeit aufweist.

Aus der DE—A—2 022 498 sind prosthetische Strukturen als Derivate von Kollagen bekannt, wobei diese Strukturen ein komplexes partielles Salz von Kollagen mit einem mehrwertigen Metallion und einem ionisierbaren Säurekation enthalten. Dieses Material ist hart und schlagfest, wobei ein Anschmiegen an Knockenhöhlen erschwert wird.

Aus der FR—A—2 350 826 schliesslich ist ein Knochenersatzmaterial bekannt, in welchem unter anderem natürliches Kollagen vermischt mit Kalziumphosphat verwendet wird. Abgesehen von den immunologischen Abwehrreaktionen, die das natürliche Kollagen verursachen kann, bewirkt das Kalziumphosphat, dass das Kollagen seine gute Saugfähigkeit und seine gute Resorbierbarkeit verliert, während umgekehrt das Kalziumphosphat relativ leicht resorbiert wird, wodurch beide Komponenten während der An- und Einwachsphase des Knochens geschwächt sind.

Es ist demgegenüber Aufgabe der vorliegenden Erfindung ein Knochenersatzmaterial anzugeben, das industriell mit gleichbleibender Qualität und einwandfreier Sterilität hergestellt werden kann, und sowohl die Nachteile des autologen oder homologen Knochenersatzes vermeidet als auch natürliche Knochenbildung in spezifischer Weise besser anregt als bisher bekannte heterologe Knochenersatzmaterialien. Ein solches Knochenersatzmaterial und Verfahren zur Herstellung desselben sind in den Ansprüchen beschrieben.

Die Erfindung wird nun anhand von Ausführungsbeispielen näher erläutert werden. In Kliniken wird seit längerer Zeit blutstillendes Material in Form von Kollagen-Vliesen verwendet, wobei hier unter Kollagen das Stützeiweiss der Bindegewebe, Haut, Sehnen und Knochen verstanden wird. Dabei wird chemisch zwischen Neutralsalz- und säurelöslichem Kollagen unterschieden, nebst einer mehr oder minder unlöslichen Kollagenfaser, in der die Kollagenmoleküle fest verbunden sind. Die chemische Kollageneinheit hat ein hohes Molekulargewicht von etwa 300'000, wobei dieses makromolekulare Peptid von drei schraubenähnlichen Proteinketten gebildet wird, deren jede ein Molekulargewicht von ungefähr 95'000 hat. Das Kollagen wird vorwiegend aus Tierhaut gewonnen und bewirkt in der richtigen Zusammensetzung eine sofortige Blutgerinnung und einen guten mechanischen Wundverschluss, so dass es vorteilhaft bei Verletzungen von Organen wie beispielsweise der Leber verwendet werden kann, aber auch bei der Bedeckung von Brandwunden oder granulierenden Wunden, Vereinzelt wurde das Kollagenmaterial auch zur Substitution von Knochendefekten in der Kieferchirurgie verwendet, doch liegen über die Ergebnisse widersprüchliche Aussagen vor. Während im Falle von sogenannten ersatzstarken Lager, d.h. innerhalb einer Knochenhöhle und kleineren Spalten neben negativen auch positive Ergebnisse angeführt werden, scheint es im Falle von sogenannten ersatzschwachen Lagern, d.h. wenn das Material beispielsweise nach völliger Knochenresektion in eine Weichteilgewebelücke gelegt werden muss, mit Kollagenfasern keine nennenswerten Erfolge zu geben. In der orthopädischen Chirurgie wäre aber gerade wirksames knochenbildendes Material im Falle des ersatzschwachen Lagers erwünscht.

Um ein Knochenersatzmaterial zu erhalten, das sowohl im ersatzstarken als auch im ersatzschwachen Lager knochenbildend ist und überall eingesetzt werden kann, ist es notwendig, dem Kollagen-Vlies Substanzen beizufügen, die diese Wirkung hervorrufen beziehungsweise verstärken. Die Substanz, die dazu geeignet ist, ist die mineralische Komponente der paraplastischen Knochensubstanz, das Apatit, das vorwiegend aus hydratisiertem Tricalciumphosphat besteht. Aus vielen Versuchen ist bekannt, dass dem Apatit eine wesentliche knochenbildende Wirkung zukommt. Wesentlich ist dabei, dass das Material in besonderen porösen Formgebungen vorkommt, um die Substitution dieses Materials durch vitales Knochengewebe zu fördern.

Es ist dabei hervorzuheben, dass der blutstillende Effekt des Kollagen-Vlieses von Vorteil ist, weil ist bei den Knochenoperationen aus den feinen Knochenkanälchen meistens stark blutet und die Blutgefässe im Unterschied zum Weichteilgewebe dort nicht durch Elektrokogulation geschlossen werden können. Dazu kommt noch, dass dieser blutstillende Effekt des Kollagen-Vlieses durch den Apatitzusatz verstärkt wird, da das Apatit-Calcium Ionen abscheidet und Calciumionen bei der Blutgerinnung eine wesentliche Rolle spielen.

Das Kollagenmaterial wird in der Regel aus Tierhaut, beispielsweise Schweinehaut, durch Behandlung mit proteolytischen Enzymen gewonnen. Anschliessend wird es gereinigt und lyophilisiert sowie mit Gammastrahlen sterilisiert. Für bestimmte Zwecke kann es jedoch notwendig sein, besonders im Hinblick auf Transplantationen, das Kollagenmaterial aus Humangewebe herzustellen. Vorzugweise wird zuerst das Kollagenmaterial in Form von Vlies gepresstem Netz- oder Maschenwerk hergestellt oder in geflochtener, gewirkter oder gewebter Textilform oder als räumliches Maschengitter bzw. Wabengitter — ähnlich der natürlichen Spongiosastruktur — hergestellt. Bei der Herstellung eines Vliesnetzes kann die Vliesschicht durch eine Rasterpresswalze laufen, durch die ein Netzmuster hineingepresst wird. Bei der Herstellung des Knochenersatzmaterials ist darauf zu achten, dass eine möglichst gleichmässige Durchmischung, durch Mischung des Kollagenmaterials mit Apatit-Pulver oder -Körnern erreicht wird. Dabei stehen zwei Verfahrensvarianten offen:

1. Das in dünnen Schichten hergestellte Kollagen-Vlies wird mit Apatit-Pulver oder -Körnern bestreut und einem Pressvorgang unterzogen, wobei eine Walze durchlaufen wird, wodurch das Apatitpulver in das Maschenwerk des Vlieses eingepresst wird. Dieses Verfahren kann sowohl trocken als auch im feuchten Zustand erfolgen.

2. Das Kollagenfasermaterial kann, bevor es als Schicht ausgeformt wird, in einem Mischer mit dem Apatit-Pulver oder -Körnern versetzt werden und anschliessend einer Schichtformung und Trocknung unterzogen werden. Während im natürlichen Knochengewebe die Anteile von Kollagen und Apatit ungefähr gleich sind, können die Anteile in einem grossen Rahmen variieren und den verschiedenen Verwendungszwecken angepasst werden. Vorteilhafterweise werden diesem Material geeignete Antibiotika beigemischt, was insbesondere für die septische Knochenchirurgie wichtig ist. Das Endprodukt kann in Form von Körnern, amorphen oder geformten Stücken, Schnitzeln, Streifen oder Röhren vorliegen. Es ist auch möglich, dem Material die Form natürlicher Knochen zu geben.

Auf herkömmliche Weise hergestelltes Kollagen-Vliesmaterial ist in der Regel sehr weich, wodurch es möglich ist, dieses Material in den Knochenhöhlen in allen Buchten hineinzustopfen und wodurch es sich der Knochenoberfläche gut anlegen bzw. anpressen lässt. Allerdings ist dies von Nachteil dort, wo Defektüberbrückungen vorzunehmen sind, die eine gewisse Festigkeit haben müssen. Es ist daher anzustreben, die Struktur des Kollagen-Vlieses zu verstärken, wofür beispielsweise Kohlehydratstärke oder Fibrin verwendet werden kann. Um die Struktur noch weiter zu verstärken, kann die Kollagenmaterial-Apatit-Mischung auch auf wabenförmige Grundstrukturen beschichtet werden, wobei diese Strukturen aus Kunststoff, Textil, Metall, Aluminiumoxyd, Keramik, Kohlenfasergewebe, Kohlenstoff oder Knochenzement bestehen und gegebenenfalls die Form des zu ersetzenden Knochenteils aufweisen können.

## Patentansprüche

1. Knochenersatzmaterial, gekennzeichnet durch eine Mischung von Kollagenvlies mit Hydroxylapatit in Form von Pulver oder Körnern, wobei das Kollagen in Form eines enzymatisch behandelten, gereinigten, lyophilisierten und sterilisierten Kollagen-Vlieses vorliegt.

2. Knochenersatzmaterial nach Anspruch 1, dadurch gekennzeichnet, dass es zwecks Strukturverstärkung Kohlehydratstärke oder Fibrin enthält.

3. Knochenersatzmaterial nach Anspruch 1 oder 2, dadurch gekennzeichnet, dass es auf einer webenförmigen Grundstuktur aufgeschichtet ist.

4. Knochenersatzmaterial nach Anspruch 3, dadurch gekennzeichnet, dass die wabenförmige Grundstruktur aus Kunststoff, Textil, Metall, Keramik, Kohlenfasergewebe, Kohlenstoff oder Knochenzement gefertigt ist.

5. Knochenersatzmaterial nach Anspruch 1 oder 2, dadurch gekennzeichnet, dass es die Form von Körnern, amorphen oder geformten Stücken, Schnitzeln, Streifen oder Röhren aufweist.

6. Knochenersatzmaterial nach einem der Ansprüche 1—4, dadurch gekennzeichnet, dass es die Form natürlicher Knochen aufweist.

7. Knochenersatzmaterial nach einem der Ansprüche 1—6, dadurch gekennzeichnet, dass es Antibiotika enthält.

8. Verfahren zur Herstellung von Knochenersatzmaterial nach Anspruch 1, dadurch gekennzeichnet, dass dünne Schichten des enzymatisch behandelten, gereinigten, lyophilisierten und sterilisierten Kollagenvlieses mit Hydroxylapatit-Pulver oder -Körnern bestreut werden und im trockenen oder feuchten Zustand einem Pressvorgang unterzogen werden.

9. Verfahren zur Herstellung von Knochenersatzmaterial nach Anspruch 1, dadurch gekennzeichnet, dass vor dem Trocken und Ausformen des Kollagenvlieses Hydroxylapatit-Pulver oder -Körner beigemischt wird.

## Revendications

1. Matériau de remplacement des os, caractérisé par un mélange de nappes de fibres de collagène avec de l'hydroxylapatite sous forme de poudre ou de grains, le collagène étant présent sous forme de fibres de collagène traitées aux enzymes, purifiées, lyophilisées et stérilisées.

2. Matériau de remplacement des os selon la revendication 1, caractérisé en ce qu'il contient un amidon de glucide ou de la fibrine.

3. Matériau de remplacement des os selon la revendication 1 ou 2, caractérisé en ce qu'il est étendu sur une structure de base alvéolée.

4. Matériau de remplacement des os selon la revendication 3, caractérisé en ce que la structure de base alvéolée est fabriquée en matériau synthétique, textile, métal, céramique, tissu de fibres de carbone, carbone ou ciment des os.

5. Matériau de remplacement des os selon la revendication 1 ou 2, caractérisé en ce qu'il est en forme de grains, de parties amorphes ou formées, de copeaux, de bandes ou de tubes.

6. Matériau de remplacement des os selon l'une des revendications 1—4, caractérisé en ce qu'il est en forme d'os naturels.

7. Matériau de remplacement des os selon l'une des revendications 1—6, caractérisé en ce qu'il contient des antibiotiques.

8. Procédé de fabrication d'un matériau de remplacement des os selon la revendication 1, caractérisé en ce que des couches minces de nappes de fibres de collagène traitées aux enzymes, purifiées, lyophilisées et stérilisées et de poudre ou de grains d'hydroxylapatite sont étendues et soumises en condition sèche ou humide à un processus de moulage.

9. Procédé de fabrication d'un matériau de remplacement des os selon la revendication 1, caractérisé en ce que de la poudre ou des grains d'hydroxylapatite sont mélangés avant le séchage et la mise en forme des nappes de fibres de collagène.

## Claims

1. A bone replacement material, characterized by a mixture of collagen fleeces with hydroxylapatite in form of powder of grains, the collagen being present in form of fleeces of collagen treated by enzymes, purified, lyophilized and sterilized.

2. A bone replacement material, characterized in that it comprises a carbohydrate starch or fibrin.

3. A bone replacement material according to claim 1 or 2, characterized in that it is piled up on an alveolate ground structure.

4. A bone replacement material according to claim 3, characterized in that the alveolate ground structure is manufactured from plastic, textile, metal, ceramics, carbon fiber tissues, carbon or bone cement.

5. A bone replacement material according to claim 1 or 2, characterized in that it is in form of grains, amorphous or formed parts, chips, stripes or tubes.

6. A bone replacement material according to one of the claims 1—4, characterized in that it has the shape of natural bones.

7. A bone replacement material according to one of the claims 1—6, characterized in that it contains antibiotics.

8. A process for producing a bone replacement material according to claim 1, characterized in that thin layers of fleeces of collagen treated by enzymes, purified, lyophilized and sterilized and of powder or grains of hydroxylapatite are piled up and submitted in dry or humid condition to a moulding cycle.

9. Process for producing a bone replacement material according to claim 1, characterized in that powder or grains of hydroxylapatite are mixed before drying and shaping of the fleeces of collagen.